# EUROPÄISCHE PATENTSCHRIFT

(11) **EP 2 653 110 B1**
(45) Veröffentlichungstag und Bekanntmachung des Hinweises auf die Patenterteilung: **13.12.2017**
(21) Anmeldenummer: 13001766.8
(22) Anmeldetag: 06.04.2013
(51) Int. Cl.: A61B 17/00, A61B 17/29

(54) **Medizinisches Instrument mit abwinkelbarem Schaft**
Medical instrument with articulatable shaft
Instrument médical doté d'une tige pliable

(30) Priorität: 18.04.2012 DE 102012007645
(43) Veröffentlichungstag der Anmeldung: 23.10.2013
(73) Patentinhaber: Karl Storz GmbH & Co. KG, 78532 Tuttlingen (DE)
(72) Erfinder: Kärcher, Daniel, 78532 Tuttlingen (DE); Stefan, Jochen, 78532 Tuttlingen (DE)

(56) Entgegenhaltungen:
- EP-A2- 1 889 579
- WO-A1-2011/056458
- DE-U1-202007 003 114
- US-A- 5 282 800
- US-A- 5 549 637

## Beschreibung

Die vorliegende Erfindung ist auf ein medizinisches Instrument mit einem abwinkelbaren Schaft und eine Gelenkeinrichtung für ein medizinisches Instrument gerichtet.

Medizinische Instrumente, insbesondere mikroinvasiv-chirurgische Instrumente, weisen eine wachsende Anzahl von Freiheitsgraden auf. Dies ermöglicht es medizinischem Personal, immer komplexere und immer mehr verschiedene Tätigkeiten mit einem einzigen Instrument auszuführen. Beispielsweise bei einem Nadelhalter oder einem anderen Greif- oder Schneidewerkzeug am distalen Ende eines langen dünnen Schafts ist für einige Anwendungen eine Abwinkelbarkeit des Werkzeugs relativ zum Schaft wünschenswert. Eine Abwinkelbarkeit bzw. Schwenkbarkeit des Werkzeugs um eine Achse senkrecht zur Längsachse des Schafts kann ein Greifen oder Schneiden in einer Richtung ermöglichen, die anderenfalls nicht erreichbar ist.

Bei mehrfach verwendbaren medizinischen Instrumenten ist eine möglichst weitgehende Zerlegbarkeit als Voraussetzung für eine vollständige Reinigung von besonderer Bedeutung. Dazu können Schraubgewinde oder Bajonettverschlüsse verwendet werden, die in der Regel auf geeignete Weise verriegelt werden. Herkömmliche Konzepte sind jedoch auf ein medizinisches Instrument mit einem abwinkelbaren Schaft nicht oder nicht ohne Weiteres übertragbar.

In US 5,282,800 ist ein chirurgisches Instrument für endoskopische Chirurgie beschrieben, das eine wiederverwendbare Handhabe und ein Einweg-Arbeitsbauteil umfasst. Eine Einweg-Schaftbaugruppe kann von proximal in ein rohrförmiges Bauteil eingeführt werden, bis ein Adapter am proximalen Ende der Schaftbaugruppe in dem rohrförmigen Bauteil liegt. Danach kann ein über ein Gelenk mit dem rohrförmigen Bauteil verbundenes Bauteil aus einer offenen Position in eine geschlossene Position geschwenkt werden, in der es die Schaftbaugruppe an der vorgesehenen Position im rohrförmigen Bauteil hält. Der Oberbegriff des unabhängigen Anspruchs 1 basiert sich auf diesem Dokument. In WO 2011/056458 A1 ist ein chirurgisches Instrument beschrieben, bei dem ähnlich wie gemäß US 5,282,800 ein Schaft mit einer Handhabe verbindbar ist. Dazu können zwei Teile der Handhabe um je ein Gelenk geschwenkt werden. In einer geschlossenen Stellung halten die beiden schwenkbaren Teile der Handhabe den Schaft in seiner vorgesehenen Position.

In EP 1 889 579 A2 ist ein zerlegbares medizinisches Rohrschaftinstrument mit einer Handhabe, einem Schaft, einem Werkzeugbetätigungselement und einem Werkzeug beschrieben.

In US 5,549,637 ist ein gelenkiges medizinisches Instrument mit mehreren Gelenken beschrieben.

In DE 20 2007 003 114 U1 ist eine medizinische Zange mit einer Bajonettverbindung zwischen einem Zangenkopf und einem Schaftrohr beschrieben.

Eine Aufgabe der vorliegenden Erfindung besteht darin, eine verbesserte Gelenkeinrichtung für ein medizinisches Instrument und ein verbessertes medizinisches Instrument zu schaffen.

Diese Aufgabe wird durch den Gegenstand des unabhängigen Anspruchs 1 gelöst.

Weiterbildungen sind in den abhängigen Ansprüchen angegeben.

Ausführungsbeispiele der vorliegenden Erfindung beruhen auf der Idee, eine Verriegelungseinrichtung an einer Kupplung in der Nähe eines Gelenks eines medizinischen Instruments so auszubilden, dass eine mechanische Verbindung mittels der Kupplung abhängig von der Winkelposition des Gelenks verriegelt oder lösbar ist. Die nachfolgend beschriebenen Varianten und Ausführungsbeispiele zeigen, dass eine Verriegelung der Kupplung in Abhängigkeit von der Winkelposition des Gelenks konstruktiv und fertigungstechnisch einfach realisierbar sein und dabei gleichzeitig eine einfache Handhabung ermöglichen kann.

Ein medizinisches Instrument umfasst einen Schaft, eine Kupplung am distalen Ende des Schafts zur lösbaren mechanischen Verbindung eines Werkzeugs mit dem distalen Ende des Schafts, eine Gelenk proximal des distalen Endes des Schafts zum Abwinkeln des distalen Endes des Schafts in eine einstellbare Winkelposition und eine Verriegelungseinrichtung an der Kupplung zum Verriegeln einer mechanischen Verbindung eines Werkzeugs mit dem distalen Ende des Schafts, wobei die Verriegelungseinrichtung so ausgebildet ist, dass die mechanische Verbindung bei allen vorbestimmten Arbeits-Winkelpositionen des Gelenks innerhalb eines vorbestimmten Arbeitsbereichs von Arbeits-Winkelpositionen verriegelt und bei einer vorbestimmten Löse-Winkelposition des Gelenks lösbar ist.

Eine Gelenkeinrichtung für ein medizinisches Instrument umfasst eine Kupplung zur lösbaren mechanischen Verbindung der Gelenkeinrichtung mit einem Werkzeug oder mit dem distalen Ende eines Schafts, eine Verriegelungseinrichtung an der Kupplung zur Verriegelung der mechanischen Verbindung und ein Gelenk, das ein Abwinkeln der Kupplung relativ zu einem von der Kupplung abgewandten Ende der Gelenkeinrichtung ermöglicht, wobei die Verriegelungseinrichtung so ausgebildet ist, dass die mechanische Verbindung bei allen vorbestimmten Arbeits-Winkelpositionen des Gelenks innerhalb eines vorbestimmten Arbeitsbereichs von Arbeits-Winkelpositionen verriegelt und bei einer vorbestimmten Löse-Winkelposition des Gelenks lösbar ist.

Das medizinische Instrument ist insbesondere ein mikroinvasiv-chirurgisches Instrument mit einem langen, dünnen, geraden oder gekrümmten, starren oder flexiblen Schaft. Am proximalen Ende des Schafts ist insbesondere eine Handhabungseinrichtung vorgesehen, die ausgebildet ist, manuell erzeugte Kräfte und Momente aufzunehmen. Der Schaft ist zum Übertragen von Kräften und Momenten von der Handhabungseinrichtung zu dem Gelenk und zu einem Werkzeug am distalen Ende des medizinischen Instruments ausgebildet. Dazu weist der Schaft insbesondere einen Außenschaft, einen Innenschaft und/oder eine Übertragungsstange und/oder andere Übertragungseinrichtungen auf Jede einzelne Übertragungseinrichtung kann starr oder biegeelastisch und zum Übertragen einer Kraft und/oder eines Drehmoments ausgebildet sein. Insbesondere ist zumindest eine Übertragungseinrichtung mit dem Gelenk gekoppelt, um eine Kraft oder ein Drehmoment zum Einstellen der Winkelposition von der Handhabungseinrichtung zu dem Gelenk zu übertragen.

Die Kupplung ist insbesondere zur lösbaren starren mechanischen Verbindung mit einem Werkzeug ausgebildet. Die Kupplung ist beispielsweise als Schraubgewinde oder als Bajonettkupplung ausgebildet. Die Gelenkeinrichtung ist insbesondere mit dem distalen Ende eines Außenschafts starr und dauerhaft bzw. unlösbar verbunden oder weist eine weitere Kupplung (Schaftkupplung) zur lösbaren und insbesondere starren mechanischen Verbindung mit einem distalen Ende eines Außenschafts auf.

Alternativ ist die Kupplung zur lösbaren starren mechanischen Verbindung mit einem distalen Ende eines Schafts ausgebildet (Schaftkupplung). Die Kupplung ist beispielsweise als Schraubgewinde oder als Bajonettkupplung ausgebildet. Die Gelenkeinrichtung ist insbesondere mit dem proximalen Ende eines Werkzeugs oder eines weiteren Schaftabschnitts starr und dauerhaft bzw. unlösbar verbunden oder weist eine weitere Kupplung (Werkzeugkupplung) zur lösbaren und insbesondere starren mechanischen Verbindung mit einem proximalen Ende eines Werkzeugs oder eines weiteren Schaftabschnitts auf.

Das Gelenk ist ausgebildet für ein Abwinkeln bzw. ein Schwenken um eine Schwenkachse, die nicht parallel zur Längsachse des Schafts und insbesondere senkrecht oder im Wesentlichen senkrecht (Winkel mindestens 60 Grad, insbesondere mindestens 80 Grad) zur Längsachse des Schafts ist. Das Gelenk kann ausgebildet sein, um ein Abwinkeln lediglich in einer einzigen Richtung bzw. in einer einzigen Ebene oder in zwei zueinander senkrechten Richtungen zu ermöglichen.

Die Schwenkachse des Gelenks kann innerhalb des Querschnitts der Gelenkeinrichtung liegen und beispielsweise durch eine Welle oder Achszapfen definiert sein. Alternativ kann die Schwenkachse außerhalb der Kontur der Gelenkeinrichtung liegen bzw. diese nicht schneiden und durch Krümmungsmittelpunkte von Gleitflächen definiert sein. Ferner kann das Gelenk als Koppelgetriebe ausgebildet sein, wobei die Schwenkachse von der Winkelposition abhängig sein kann. Alternativ kann das Gelenk als elastischer Bereich eines Schaftabschnitts ausgebildet sein, wobei die Elastizität auf der Elastizität eines Bauelements und/oder auf mehreren hintereinander angeordneten Einzelgelenken beruhen kann. Gelenk und Kupplung können unmittelbar benachbart zueinander angeordnet sein, d. h. so nah, wie es konstruktiv möglich ist. Alternativ befinden sich Gelenk und Kupplung lediglich in gegenseitiger Nähe, weisen also einen Abstand auf, der im Verhältnis zu den Gesamtabmessungen des medizinischen Instruments klein ist, beispielsweise ein Zehntel der Gesamtlänge des medizinischen Instruments. Ferner können Gelenk und Kupplung weiter voneinander beabstandet sein.

Die Verriegelungseinrichtung ist ausgebildet, um die mechanische Verbindung bei mehreren vorbestimmten Arbeits-Winkelpositionen des Gelenks zu verriegeln, insbesondere innerhalb eines (insbesondere zusammenhängenden) Arbeitsbereichs von Arbeits-Winkelpositionen. Ferner kann die Verriegelungseinrichtung so ausgebildet sein, dass die mechanische Verbindung bei mehreren vorbestimmten Löse-Winkelpositionen lösbar ist, insbesondere innerhalb eines (insbesondere zusammenhängenden) Lösebereichs von Löse-Winkelpositionen.

Die Verriegelung und Entriegelung einer mechanischen Verbindung mittels einer Kupplung in Abhängigkeit von der Winkelposition eines Gelenks, das mit der Kupplung bzw. mit deren Verriegelungseinrichtung gekoppelt ist, kann eine besonders einfache Handhabung beim Zerlegen und Zusammensetzen eines medizinischen Instruments ermöglichen. Hinsichtlich Konstruktion, Fertigung und Handhabung kann vorteilhaft sein, dass keine zusätzliche Betätigungseinrichtung zum Entriegelung der Kupplung erforderlich ist. Die hier beschriebenen Varianten und Ausführungsbeispiele zeigen teilweise überraschend einfache und robuste mechanische Lösungen.

Bei einem medizinischen Instrument oder einer Gelenkeinrichtung, wie sie hier beschrieben ist, ist die Verriegelungseinrichtung insbesondere so ausgebildet, dass die mechanische Verbindung in einem vorbestimmten Arbeitsbereich von Arbeits-Winkelpositionen des Gelenks verriegelt ist.

Der Arbeitsbereich umfasst beispielsweise Arbeits-Winkelpositionen zwischen 0° (Gelenk vollständig gestreckt) und 80° (Gelenk fast rechtwinklig abgewinkelt). Löse-Winkelpositionen können sich an einem Ende oder an beiden Enden des Arbeitsbereichs anschließen. Eine Löse-Winkelposition liegt beispielsweise bei -10° bzw. bei einer Abwinklung des Gelenks um 10° in einer zu den Arbeits-Winkelpositionen entgegengesetzten Richtung. Alternativ oder zusätzlich kann eine (weitere) Löse-Winkelposition am entgegengesetzten Ende des Arbeitsbereichs liegen, beim genannten Beispiel eines Arbeitsbereichs von 0° bis 80° beispielsweise bei einem Winkel von 90°.

Bei einem medizinischen Instrument oder einer Gelenkeinrichtung, wie sie hier beschrieben ist, kann die Verriegelungseinrichtung ausgebildet sein, um ein Herstellen und Verriegeln der mechanischen Verbindung auch in der Arbeits-Winkelposition des Gelenks zu ermöglichen.

Insbesondere kann ein Riegel vorgesehen sein, der auch in einer Arbeits-Winkelposition aufgrund seiner (beispielsweise keil- oder klinkenförmigen) Gestalt gegen die Kraft einer Feder oder eines anderen elastischen Elements in eine entriegelnde Position bewegt werden kann und bei Erreichen der vorgesehenen mechanischen Verbindung in seine verriegelnde Position zurückkehrt.

Bei einem medizinischen Instrument oder einer Gelenkeinrichtung, wie sie hier beschrieben ist, umfasst die Verriegelungseinrichtung insbesondere einen in axialer Richtung der Kupplung bewegbaren Riegel, wobei in einer vorbestimmten verriegelnden Position des Riegels die mechanische Verbindung verriegelt und in einer vorbestimmten entriegelnden Position des Riegels die mechanische Verbindung lösbar ist.

Der Riegel wird insbesondere durch eine Feder oder durch eine andere elastische Einrichtung in die verriegelnde Position bewegt. Der Riegel ist mit dem Gelenk derart gekoppelt, dass der Riegel in seine entriegelnde Position bewegt wird, wenn das Gelenk in die Löse-Winkelposition bewegt wird, und dass der Riegel seine vorbestimmte verriegelnde Position oder eine von mehreren verriegelnden Positionen einnimmt, wenn das Gelenk sich in seiner Arbeits-Winkelposition oder in einer von mehreren Arbeits-Winkelpositionen befindet.

Bei einem medizinischen Instrument oder einer Gelenkeinrichtung mit einem Riegel, wie sie hier beschrieben ist, sind insbesondere die Kupplung eine Werkzeugkupplung zur lösbaren mechanischen Verbindung der Gelenkeinrichtung mit einem Werkzeug und der Riegel an einem die Kupplung umfassenden distalen Teil der Gelenkeinrichtung angeordnet und geführt, wobei bei der vorbestimmten Löse-Winkelposition des Gelenks der Riegel an einem proximalen Teil der Gelenkeinrichtung, der mit einem Schaft verbunden oder verbindbar ist, anliegt und von diesem in die entriegelnde Position geschoben ist.

Eine Betätigung eines am distalen Teil der Gelenkeinrichtung angeordneten und geführten Riegels durch einen proximalen Teil der Gelenkeinrichtung kann eine hinsichtlich Konstruktion und Fertigung besonders einfache und robuste Verriegelung der Kupplung ermöglichen. Dies gilt insbesondere, wenn die durch das Gelenk definierte Schwenkachse außerhalb der Gelenkeinrichtung liegt.

Bei einem medizinischen Instrument oder einer Gelenkeinrichtung, wie sie hier beschrieben ist, ist die Kupplung insbesondere eine Bajonettkupplung und umfasst eine Nut zur Aufnahme einer Knagge an einem proximalen Ende eines Werkzeugs.

Alternativ kann die Kupplung eine Bajonettkupplung sein und eine Knagge, die zur Aufnahme in einer Nut am proximalen Ende eines Werkzeugs vorgesehen ist, umfassen.

Die Nut ist insbesondere L-förmig mit einem axialen Abschnitt (der sich in axialer Richtung oder im Wesentlichen in axialer Richtung erstreckt) und einem umfänglichen Abschnitt (der sich in umfänglicher Richtung oder im Wesentlichen in umfänglicher Richtung erstreckt).

Bei einer Gelenkeinrichtung, wie sie hier beschrieben ist, ist die Kupplung insbesondere eine Werkzeugkupplung zur lösbaren mechanischen Verbindung der Gelenkeinrichtung mit einem Werkzeug, wobei die Gelenkeinrichtung ferner eine Schaftkupplung zur lösbaren mechanischen Verbindung der Gelenkeinrichtung mit einem Schaft und eine Schaft-Verriegelungseinrichtung umfasst, wobei die Schaft-Verriegelungseinrichtung ausgebildet ist, um die mechanische Verbindung der Gelenkeinrichtung mit einem Schaft mittels der Schaftkupplung zu verriegeln.

Die Gelenkeinrichtung kann somit (insbesondere an entgegengesetzten Enden) eine Werkzeugkupplung zur lösbaren mechanischen Verbindung mit einem Werkzeug und eine Schaftkupplung zur lösbaren mechanischen Verbindung mit einem Schaft umfassen. Dies kann eine weitgehende Zerlegung und eine besonders vollständige Reinigung eines medizinischen Instruments mit der Gelenkeinrichtung ermöglichen.

Die Schaft-Verriegelungseinrichtung ist insbesondere derart mit dem Gelenk gekoppelt, dass die Schaftkupplung bei einer Schaftlöse-Winkelposition des Gelenks entriegelt ist.

Die Löse-Position des Gelenks, bei der die Werkzeugkupplung entriegelt ist, und die Schaftlöse-Winkelposition, bei der die Schaftkupplung entriegelt ist, können identisch, benachbart, aneinander angrenzend oder voneinander beabstandet sein. Insbesondere können die Löse-Winkelposition und die Schaftlöse-Winkelposition an entgegengesetzten Enden des Arbeitsbereichs bzw. des Bereichs der Arbeits-Winkelpositionen angeordnet sein. Wenn die Löse-Winkelposition und die Schaftlöse-Winkelposition identisch sind, können die Werkzeugkupplung und die Schaftkupplung gleichzeitig entriegelt werden. Wenn die Löse-Winkelposition und die Schaftlöse-Winkelposition benachbart sind bzw. aneinander angrenzen, können die Werkzeugkupplung und die Schaftkupplung unmittelbar nacheinander entriegelt werden. Wenn die Löse-Winkelposition und die Schaftlöse-Winkelposition an entgegengesetzten Enden des Arbeitsbereichs und damit weit voneinander beabstandet liegen, kann eine unbeabsichtigte Entriegelung von einer der beiden Kupplungen besonders wirksam verhindert werden.

Eine Gelenkeinrichtung mit einer Schaftkupplung, wie sie hier beschrieben ist, umfasst ferner insbesondere eine Übertragungseinrichtung, die mit dem Gelenk gekoppelt ist, zum Übertragen einer Kraft vom proximalen Ende eines medizinischen Instruments zu dem Gelenk, und zum Einstellen einer Winkelposition des Gelenks vom proximalen Ende des medizinischen Instruments aus, wobei die Schaft-Verriegelungseinrichtung mit der Übertragungseinrichtung gekoppelt ist.

Die Übertragungseinrichtung umfasst insbesondere einen Innenschaft oder eine Übertragungsstange. Die Übertragungseinrichtung ist insbesondere in einem Außenschaft parallel zu dessen Längsachse verschiebbar. Insbesondere nimmt das Gelenk in einer proximalen Position der Übertragungseinrichtung eine gestreckte Winkelposition und bei einer distalen Position der Übertragungseinrichtung eine abgewinkelte Winkelposition ein. Die Schaft-Verriegelungseinrichtung ist insbesondere starr oder lediglich um die Längsachse der Übertragungseinrichtung rotierbar mit der Übertragungseinrichtung verbunden.

Bei einer Gelenkeinrichtung, wie sie hier beschrieben ist, bei der die Kupplung keine Werkzeugkupplung ist, ist die Kupplung insbesondere eine Schaftkupplung zur lösbaren mechanischen Verbindung der Gelenkeinrichtung mit einem Schaft, wobei ein Riegel an einem die Kupplung umfassenden proximalen Teil der Gelenkeinrichtung angeordnet und geführt ist, und wobei bei der vorbestimmten Löse-Winkelposition des Gelenks der Riegel an einem distalen Teil der Gelenkeinrichtung, der mit einem Werkzeug verbunden oder verbindbar ist, anliegt und von dem distalen Teil der Gelenkeinrichtung in eine entriegelnde Position geschoben ist.

Ein medizinisches Instrument umfasst eine Gelenkeinrichtung, wie sie hier beschrieben ist.

Das medizinische Instrument umfasst insbesondere ferner einen Schaft, dessen distales Ende mit der Gelenkeinrichtung verbindbar oder verbunden ist, und eine Handhabungseinrichtung, die mit dem proximalen Ende des Schafts lösbar mechanisch koppelbar ist, wobei die vorbestimmte Löse-Winkelposition des Gelenks nicht erreichbar ist, wenn die Handhabungseinrichtung mit dem proximalen Ende des Schafts gekoppelt ist.

Ein Blockieren der Löse-Winkelposition und im Falle zweier Kupplungen von (gleichen oder unterschiedlichen) Löse-Winkelpositionen durch die Handhabungseinrichtung kann ermöglichen, dass ein mehrfach zerlegbares medizinisches Instrument allein nach Betätigen einer Entriegelungs-Betätigungseinrichtung an der Handhabungseinrichtung und Lösen des Schafts von der Handhabungseinrichtung vollständig zerlegbar ist. Dies ermöglicht ein besonders einfaches und schnelles Zerlegen des medizinischen Instruments nach kurzer Schulung und kann so die erforderlichen Personalkosten reduzieren.

### Kurzbeschreibung der Figuren

Nachfolgend werden Ausführungsformen anhand der beigefügten Figuren näher erläutert. Es zeigen:
Figur 1 eine schematische Darstellung eines medizinischen Instruments;
Figur 2 eine schematische axonometrische Darstellung eines Werkzeugs;
Figur 3 eine weitere schematische axonometrische Darstellung des Werkzeugs aus Figur 2;
Figur 4 eine schematische axonometrische Darstellung einer Gelenkeinrichtung;
Figur 5 eine weitere schematische axonometrische Darstellung der Gelenkeinrichtung aus Figur 4;
Figur 6 eine weitere schematische axonometrische Darstellung der Gelenkeinrichtung aus den Figuren 4 und 5;
Figur 7 eine weitere schematische axonometrische Darstellung der Gelenkeinrichtung aus den Figuren 4 bis 6;
Figur 8 eine weitere schematische axonometrische Darstellung der Gelenkeinrichtung aus den Figuren 4 bis 7;
Figur 9 eine schematische axonometrische Darstellung eines distalen Endes eines Schafts;
Figur 10 eine weitere schematische axonometrische Darstellung des distalen Endes eines Schafts aus Figur 9;
Figur 11 eine weitere schematische Darstellung der Gelenkeinrichtung aus den Figuren 4 bis 8;
Figur 12 eine weitere schematische Darstellung der Gelenkeinrichtung aus den Figuren 4 bis 8 und 11;
Figur 13 eine weitere schematische Darstellung der Gelenkeinrichtung aus den Figuren 4 bis 8, 11 und 12.

### Beschreibung der Ausführungsformen

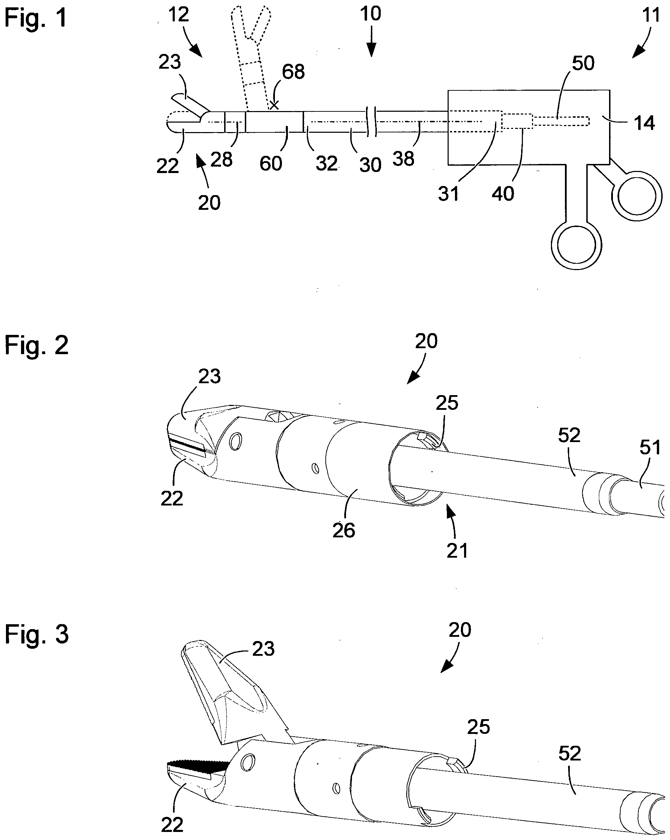

Figur 1 zeigt eine schematische Darstellung eines medizinischen Instruments 10 mit einem proximalen Ende 11 und einem distalen Ende 12. Das medizinische Instrument 10 umfasst am proximalen Ende 11 eine Handhabungseinrichtung 14 und am distalen Ende 12 ein Werkzeug 20. Die Handhabungseinrichtung 14 umfasst mehrere Griffteile, die zumindest teilweise relativ zueinander bewegbar sind. Ferner kann die Handhabungseinrichtung 14 weitere Betätigungseinrichtungen umfassen, die manuell bewegbar sind.

Die Handhabungseinrichtung 14 und das Werkzeug 20 sind durch einen Außenschaft 30 und eine Gelenkeinrichtung 60 miteinander mechanisch verbunden. Im Außenschaft 30 sind ein Innenschaft 40 und eine Übertragungsstange 50 konzentrisch angeordnet. In Figur 1 sind nur jeweils die proximalen Enden des Innenschafts 40 und der Übertragungsstange 50 angedeutet. Der Innenschaft 40 und die Übertragungsstange 50 sind unabhängig voneinander jeweils parallel zur Längsachse 38 des Außenschafts verschiebbar und/oder um die Längsachse 38 rotierbar.

Bei konzentrischer Anordnung von Außenschaft 30, Innenschaft 40 und Übertragungsstange 50 ist die Längsachse 38 des Außenschafts 30 gleichzeitig Längsachse von Innenschaft 40 und Übertragungsstange. Außenschaft 30, Innenschaft 40 und Übertragungsstange 50 können jeweils rotationssymmetrisch zur Längsachse 38 sein. Wenn der Außenschaft 30 (und mit ihm Innenschaft 40 und Übertragungsstange 50) abweichend von der Darstellung in Figur 1 gekrümmt sind, ist mit der Längsachse 38 nachfolgend die Längsachse des Außenschafts 30 an seinem distalen Ende 32 gemeint.

Das Werkzeug 20 umfasst ein feststehendes Maulteil 22 und ein um eine Schwenkachse senkrecht zur Zeichenebene der Figur 1 schwenkbares Maulteil 23. Die Gelenkeinrichtung 60 ermöglicht ein Schwenken des gesamten Werkzeugs 20 mit beiden Maulteilen 22, 23 um eine Schwenkachse 68 senkrecht zur Zeichenebene der Figur 1 bis zu einer Position, die in Figur 1 in gestrichelten Linien angedeutet ist. Unabhängig davon, d. h. insbesondere in jeder Winkelposition der Gelenkeinrichtung 60, ist das Werkzeug 20 um seine Längsachse 28 rotierbar. Die Längsachse 28 des Werkzeugs 20 ist nur in der in Figur 1 in durchgezogenen Linien dargestellten gestreckten Winkelposition der Gelenkeinrichtung 60 parallel zur Längsachse 38 des Außenschafts 30.

Die Handhabungseinrichtung 14 ist ausgebildet, um von medizinischem Personal manuell erzeugte bzw. auf Griffteile oder andere Betätigungseinrichtungen an der Handhabungseinrichtung 14 ausgeübte Kräfte und Momente auf den Außenschaft 30, den Innenschaft 40 und die Übertragungsstange 50 zu übertragen. Insbesondere sind ein oder mehrere bewegbare Griffteile, Drehräder oder andere Betätigungseinrichtungen an der Handhabungseinrichtung 14 derart mit dem Innenschaft 40 und der Übertragungsstange 50 gekoppelt, dass der Innenschaft 40 und die Übertragungsstange 50 durch Betätigen der Betätigungseinrichtungen parallel zur Längsachse 38 verschoben oder um die Längsachse 38 rotiert werden können.

In Figur 1 nicht dargestellte distale Enden des Innenschafts 40 und der Übertragungsstange 50 sind mit dem Werkzeug 20 und der Gelenkeinrichtung 60 gekoppelt. Bei den nachfolgend anhand der Figuren 2 bis 13 dargestellten Beispielen sind insbesondere der Innenschaft 40 mit der Gelenkeinrichtung 60 und die Übertragungsstange 50 mit dem Werkzeug 20 gekoppelt. Durch eine Verschiebung des Innenschafts 40 parallel zur Längsachse 38 können die Gelenkeinrichtung 60 verstellt und das Werkzeug 20 zwischen der in durchgezogenen Linien dargestellten gestreckten Position und der in gestrichelten Linien dargestellten abgewinkelten Position stufenlos geschwenkt werden. Durch eine Verschiebung der Übertragungsstange 50 parallel zur Längsachse 38 kann das schwenkbare Maulteil 23 zum feststehenden Maulteil 22 hin und von diesem weg geschwenkt werden. Durch eine Rotation der Übertragungsstange 50 um die Längsachse 38 kann das Werkzeug 20 um dessen Längsachse 28 rotiert werden. Die Übertragungsstange 50 ist zumindest im Bereich der Gelenkeinrichtung 60 biegeelastisch und über ihre gesamte Länge torsionssteif ausgebildet. Wenn der Außenschaft 30 abweichend von der Darstellung in Figur 1 gekrümmt oder biegeelastisch ist, sind der Innenschaft 40 und die Übertragungsstange 50 insbesondere zumindest in gekrümmten oder elastischen Bereichen des Außenschafts 30 ebenfalls biegeelastisch ausgebildet.

Nachfolgend sind anhand der Figuren 2 bis 13 Ausführungsbeispiele des Werkzeugs 20, des Außenschafts 30 und der Gelenkeinrichtung 60 dargestellt, die sowohl zur Bildung des oben anhand der Figur 1 dargestellten medizinischen Instruments 10 als auch zur Bildung eines medizinischen Instruments mit Merkmalen und Eigenschaften, die von den oben anhand der Figur 1 dargestellten abweichen, geeignet sein können.

Figur 2 zeigt eine schematische axonometrische Darstellung eines Werkzeugs 20 mit einem proximalen Ende 21 und distalen Ende, das von einem feststehenden Maulteil 22 und einem schwenkbaren Maulteil 23 gebildet wird. Das Werkzeug 20 weist am proximalen Ende 21 zwei einander gegenüberliegende Klauen bzw. Knaggen 25 auf, die jeweils radial nach innen ragen. Die Knaggen 25 bilden eine Kupplung zur lösbaren mechanischen Verbindung des Werkzeugs 20 mit dem distalen Ende einer Gelenkeinrichtung 60 (vgl. Figur 1). Am proximalen Ende 21 weist das Werkzeug 20 eine Hülse 26 auf, die unter anderem die Knaggen 25 kreismantelförmig umgibt.

Das Werkzeug 20 ist mit dem distalen Ende der Übertragungsstange 50 (vgl. Figur 1) verbunden. In Figur 2 sind ein starrer proximaler Abschnitt 51 und ein elastischer distaler Abschnitt 52 der Übertragungsstange erkennbar. Der starre proximale Abschnitt 51 nimmt den größten Teil der Länge der Übertragungsstange ein. Der elastische distale Abschnitt 52 ist zur Anordnung im Bereich der Gelenkeinrichtung 60 (vgl. Figur 1) vorgesehen. Dazu ist der elastische distale Abschnitt 52 der Übertragungsstange biegeelastisch, jedoch für die Übertragung von Zug- und Druckkräften in Längsrichtung und von Drehmomenten steif ausgebildet.

Die Übertragungsstange, insbesondere ihr elastischer distaler Abschnitt 52 ist mit dem schwenkbaren Maulteil 23 des Werkzeugs 20 derart wirksam gekoppelt, dass eine Verschiebung der Übertragungstange in ihrer Längsrichtung mit einer öffnenden oder schließenden Schwenkbewegung des schwenkbaren Maulteils 23 relativ zum feststehenden Maulteil 22 gekoppelt ist. Ferner sind die Maulteile 22, 23 relativ zum proximalen Ende 21, den Knaggen 25 und der Hülse 26 des Werkzeugs 20 um die Längsachse 28 (vgl. Figur 1) rotierbar. Eine Rotation der Übertragungsstange 51, 52 um ihre Längsachse bewirkt eine entsprechende Rotation der Maulteile 22, 23.

Figur 3 zeigt eine weitere schematische axonometrische Darstellung des Werkzeugs 20 aus Figur 2 aus einer ähnlichen Perspektive bzw. von einem ähnlichen Beobachterstandpunkt aus. Die Darstellung in Figur 3 unterscheidet sich von der Darstellung in Figur 2 insbesondere dadurch, dass das schwenkbare Maulteil 23 in einer offenen bzw. geöffneten Position gezeigt ist. Ein weiterer Unterschied zwischen den Figuren 2 und 3 besteht darin, dass die Hülse 26 in Figur 3 nicht gezeigt ist. Dadurch sind die Knaggen 25 besser erkennbar.

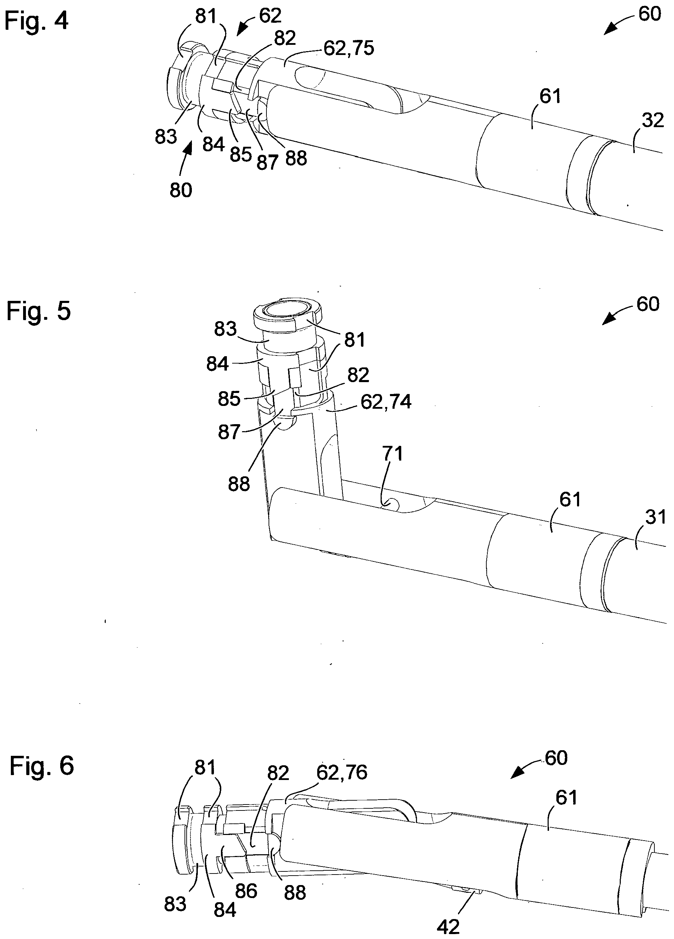

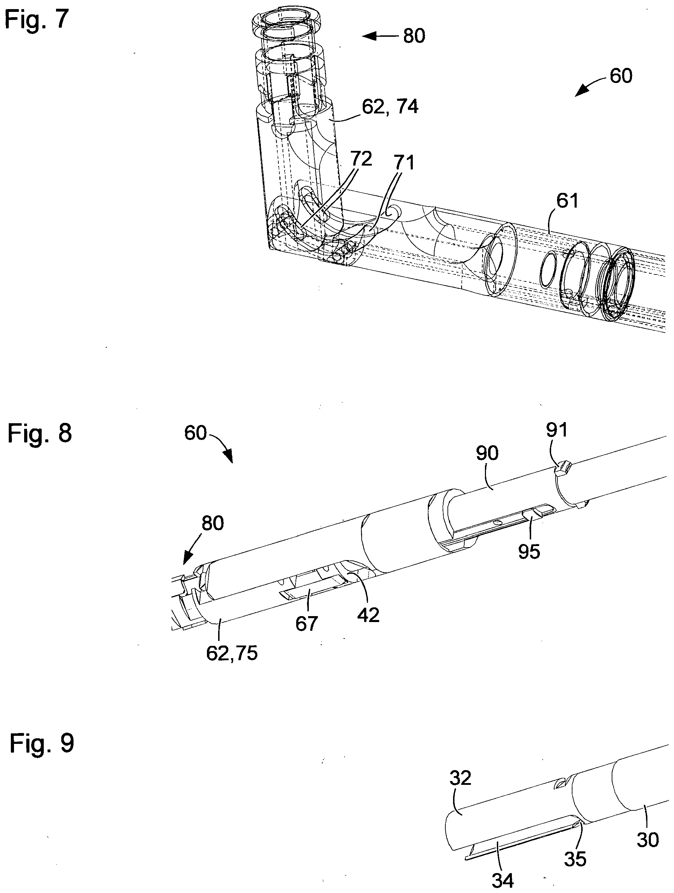

Figur 4 zeigt eine schematische axonometrische Darstellung einer Gelenkeinrichtung 60 mit einem proximalen Teil 61 und einem distalen Teil 62. Wie unten mit Bezug auf die Figuren 5 bis 8 dargestellt ist, ist der distale Teil 62 relativ zum proximalen Teil 61 der Gelenkeinrichtung um eine Schwenkachse 68 (vgl. Figur 1) schwenkbar. Der proximale Teil 61 der Gelenkeinrichtung 60 ist mit dem distalen Ende 32 eines Außenschafts 30 lösbar starr mechanisch verbindbar. Diese mechanische Kopplung ist unten mit Bezug auf die Figuren 8 bis 13 beschrieben.

Am distalen Ende des distalen Teils 62 der Gelenkeinrichtung 60 ist eine Kupplung 80 vorgesehen. Die Kupplung 80 umfasst zwei einander gegenüberliegende L-förmige Nuten, von denen in Figur 4 nur eine dem Betrachter zugewandt und deshalb sichtbar ist. Die L-förmigen Nuten weisen jeweils einen axialen Abschnitt 81 und einen umfänglichen Abschnitt 82 auf, die so angeordnet und ausgebildet sind, dass jede L-förmige Nut 81, 82 eine Knagge 25 des Werkzeugs 20 (vgl. Figur 2) aufnehmen kann.

Die Kupplung 80 umfasst ferner einen in axialer Richtung bewegbaren ringförmigen Schieber 84, der von einer Feder 83 in der in Figur 4 gezeigten Position gehalten wird. Am Schieber 84 sind zwei sich in axialer Richtung nach proximal erstreckende Riegel 85 vorgesehen, von denen in Figur 4 nur einer dem Betrachter zugewandt und deshalb sichtbar ist. Der Riegel 85 weist an seinem proximalen Ende eine dreieckige bzw. keilförmige bzw. klinkenförmige Gestalt auf und ragt bei der in Figur 4 gezeigten Position des Schiebers 84 in den umfänglichen Abschnitt 82 der L-förmigen Nut. Der Riegel 85 ist über einen dünnen, am Grund des umfänglichen Abschnitts 82 und außerhalb des Querschnitts des umfänglichen Abschnitts 82 der L-förmigen Nut angeordneten Steg 87 mit einem Nocken 88 starr verbunden, insbesondere einstückig ausgebildet.

Die Gelenkeinrichtung 60, insbesondere ihr distaler Teil 62, ist in Figur 4 in einer gestreckten Arbeits-Winkelposition 75 dargestellt, die der in Figur 1 in durchgezogenen Linien dargestellten Position der Gelenkeinrichtung und des Werkzeugs 20 entspricht. Wenn die mit dem Werkzeug 20 verbundene Übertragungsstange 51, 52 in einen in Figur 4 nicht sichtbaren zentrischen Kanal in der Gelenkeinrichtung 60 und im Schaft eingeführt ist, können die Knaggen 25 des in den Figuren 2 und 3 gezeigten Werkzeugs 20 von distal nach proximal durch die axialen Abschnitte 81 der L-förmigen Nuten bis zu deren umfänglichen Abschnitten 82 geführt werden. Bei einer anschließenden Rotationsbewegung des Werkzeugs 20 relativ zur Gelenkeinrichtung 60 und insbesondere zur Kupplung 80 können die Knaggen 25 in den umfänglichen Abschnitten 82 der L-förmigen Nuten bewegt werden. Aufgrund der dreieckigen Gestalt der Riegel 85 wird der Schieber 84 zusammen mit den Riegeln 85 und den Nocken 88 gegen die Kraft der Feder 83 nach distal verschoben bis die Knaggen 25 an den Riegeln 85 vollständig vorbei bewegt sind und der Schieber 84 mit den Riegeln 85 und den Nocken 88 durch die Kraft der Feder 83 in seine in Figur 4 gezeigte Position zurückkehrt. Die Riegel 85 halten dann formschlüssig die Knaggen 25 in der erreichten Position und verriegeln so die starre mechanische Verbindung zwischen dem Werkzeug 20 und der Gelenkeinrichtung 60 bzw. deren Kupplung 80.

Figur 5 zeigt eine weitere schematische axonometrische Darstellung der Gelenkeinrichtung 60 aus Figur 4. Die Darstellung in Figur 5 unterscheidet sich von der Darstellung in Figur 4 dadurch, dass die Gelenkeinrichtung 60, insbesondere der distale Teil 62 der Gelenkeinrichtung 60 in einer abgewinkelten Arbeits-Winkelposition 74 gezeigt ist, die näherungsweise der in Figur 1 in gestrichelten Linien gezeigten Winkelposition der Gelenkeinrichtung und des Werkzeugs 20 entspricht. An der in Figur 5 teilweise sichtbaren Innenseite des gabelförmigen proximalen Teils 61 der Gelenkeinrichtung 60 ist ein Ende einer gekrümmten Nut 71 erkennbar. Die insbesondere kreisbogenförmige Nut 71 bildet zusammen mit einem in Figur 5 nicht sichtbaren Steg am distalen Teil 62 der Gelenkeinrichtung 60, der in die Nut 71 eingreift, und zusammen mit einer weiteren Nut und einem weiteren Steg, die spiegelsymmetrisch angeordnet sind, das eigentliche Gelenk der Gelenkeinrichtung 60. Die Krümmungsmittelpunkte der Nuten 71 definieren die Lage der Schwenkachse 68 (vgl. Figur 1). Bei dem hier dargestellten Beispiel liegt die Schwenkachse 68 außerhalb des Querschnitts bzw. der Kontur der Gelenkeinrichtung 60, d. h. die Schwenkachse 68 schneidet die Gelenkeinrichtung 60 nicht. Das Gelenk ist unten mit Bezug auf die Figur 7 dargestellt.

Der Nocken 88 am Schieber 84 liegt bei der in Figur 4 gezeigten gestreckten Arbeits-Winkelposition 75 des distalen Teils 62 der Gelenkeinrichtung 60 am proximalen Teil 61 der Gelenkeinrichtung 60 an oder weist einen geringen Abstand von diesem auf. Bei der in Figur 5 gezeigten abgewinkelten Arbeits-Winkelposition 74 des distalen Teils 62 der Gelenkeinrichtung 60 ist der Nocken 88 am Schieber 84 vom proximalen Teil 61 der Gelenkeinrichtung beabstandet. In beiden Fällen nimmt der Schieber 84 aufgrund der Wirkung der Feder 83 seine äußerst proximale Position ein. Bei dieser äußerst proximalen Position des Schiebers 84 nehmen die Riegel 85 ihre bereits oben anhand der Figur 4 beschriebenen verriegelnden Positionen ein, wobei jeder Riegel 85 teilweise in den umfänglichen Abschnitt 82 von einer der L-förmigen Nuten ragt.

Figur 6 zeigt eine weitere schematische axonometrische Darstellung der Gelenkeinrichtung 6 aus den Figuren 4 und 5. Die Darstellung in Figur 6 unterscheidet sich von den Darstellungen in den Figuren 4 und 5 dadurch, dass die Gelenkeinrichtung, insbesondere der distale Teil 62 der Gelenkeinrichtung 60, eine Löse-Winkelposition 76 einnimmt. Bezogen auf die in Figur 4 gezeigte gestreckte Arbeits-Winkelposition 75 liegt die Löse-Winkelposition 76 der in Figur 5 gezeigten abgewinkelten Arbeits-Winkelposition 74 gegenüber. Anders ausgedrückt liegt die in Figur 4 gezeigte gestreckte Arbeits-Winkelposition 75 zwischen der in Figur 5 gezeigten abgewinkelten Arbeits-Winkelposition 74 und der in Figur 6 gezeigten Löse-Winkelposition 76.

Aufgrund der Anordnung der Schwenkachse 68 (vgl. Figur 1) beabstandet von den Geraden, auf denen die Nocken 88 bewegbar sind, liegen die Nocken 88 bei der Löse-Winkelposition 76 des distalen Teils 62 der Gelenkeinrichtung 60 an einer distalen Stirnfläche des proximalen Teils 61 der Gelenkeinrichtung an, und der Schieber 84 ist gegen die Kraft der Feder 83 in eine distale Position verschoben. Bei dieser distalen Position des Schiebers 84 nehmen die Riegel entriegelnde Positionen 86 ein. In der entriegelnden Position 86 ragen die Riegel nicht in den umfänglichen Abschnitt 82 der L-förmigen Nut an der Kupplung 80. Knaggen 25 eines Werkzeugs 20 (vgl. Figuren 2 und 3) können deshalb in den umfänglichen Abschnitten 82 und damit in den gesamten L-förmigen Nuten frei bewegt werden. Deshalb kann bei der in Figur 6 gezeigten Löse-Winkelposition des distalen Teils 62 der Gelenkeinrichtung 60 eine mechanische Verbindung zwischen einem Werkzeug 20 und der Gelenkeinrichtung 60 gelöst bzw. aufgehoben bzw. getrennt werden.

In Figur 6 ist ein Teil eines distalen Endes 42 eines Innenschafts 40 (vgl. Figur 1) erkennbar, dessen Funktion unten mit Bezug auf die Figuren 8 und 11 bis 13 näher beschrieben ist.

Figur 7 zeigt eine weitere schematische axonometrische Darstellung der Gelenkeinrichtung 60 aus den Figuren 4 bis 6. Die Darstellung in Figur 7 entspricht insbesondere hinsichtlich der gezeigten Arbeits-Winkelposition 74 des distalen Teils 62 der Gelenkeinrichtung 60 und hinsichtlich der Perspektive bzw. dem Beobachterstandpunkt der Figur 5. Die Darstellung in Figur 7 unterscheidet sich von der Darstellung in Figur 5 insbesondere dadurch, dass alle Elemente bzw. Bauteile der Gelenkeinrichtung 60 transparent dargestellt sind. Sichtbare Konturen, Kanten, Ränder und Grenzlinien zwischen Flächen unterschiedlicher Krümmung sind durch durchgezogene Linien, unsichtbare durch gestrichelte Linien dargestellt.

In Figur 7 sind die bereits oben erwähnten kreisbogenförmigen Nuten 71 an den Innenseiten der distalen Enden des gabelförmigen proximalen Teils 61 der Gelenkeinrichtung 60 erkennbar. Ferner sind Stege 72 an den Außenseiten des distalen Teils 62 der Gelenkeinrichtung 60 erkennbar, die in je eine der Nuten 71 eingreifen. Die gekrümmten Nuten 71 und die Stege 72 bilden das eigentliche Gelenk der Gelenkeinrichtung 60. Die Krümmungsmittelpunkte der an einander anliegenden Gleitflächen in den Nuten 71 und an den Stegen 72 definieren die Position und Orientierung der Schwenkachse.

Figur 8 zeigt eine weitere schematische axonometrische Darstellung der Gelenkeinrichtung aus den Figuren 4 bis 7. Die Darstellung in Figur 8 entspricht hinsichtlich der gezeigten Arbeits-Winkelposition 75 des distalen Teils 62 der Gelenkeinrichtung 60 der Darstellung in Figur 4. Die Darstellung in Figur 8 unterscheidet sich von der Darstellung in Figur 4 durch eine andere Perspektive bzw. einen anderen Beobachterstandort. Insbesondere ist ein etwas größerer Teil des auch in Figur 6 teilweise sichtbar distalen Endes 42 des Innenschafts 40 sichtbar. Das gekröpfte distale Ende 42 des Innenschafts 40 ist mittels eines Pleuels 76 mit dem distalen Teil 62 der Gelenkeinrichtung 60 gekoppelt bzw. gelenkig verbunden. Eine axiale bzw. translatorische Bewegung des Innenschafts 40 parallel zur Längsachse 38 (vgl. Figur 1) ist vermittels des Pleuels 67 mit einer Schwenkbewegung des distalen Teils 62 der Gelenkeinrichtung 60 gekoppelt.

In Figur 8 ist ferner eine Schaftkupplung 90 am proximalen Ende des proximalen Teils 61 der Gelenkeinrichtung 60 erkennbar. Die Schaftkupplung 90 umfasst an einem proximalen Rand eines rohrförmigen Abschnitts zwei einander gegenüberliegende, nach radial außen ragende Klauen bzw. Knaggen 91. Ferner ist in einem axialen Schlitz in der Schaftkupplung 90 ein Riegel verschiebbar, der in Figur 8 in einer verriegelnden Position 95 gezeigt ist. Der Riegel 95 ist insbesondere starr mit dem Innenschaft 40 verbunden und mit diesem zusammen in axialer Richtung verschiebbar.

Figur 9 zeigt eine schematische axonometrische Darstellung eines distalen Endes 32 eines Außenschafts 30. Das distale Ende 32 des Außenschafts 30 ist als Bajonettkupplung mit zwei einander gegenüberliegenden L-förmigen Schlitzen ausgebildet. Einer der beiden L-förmigen Schlitze ist in Figur 9 weitgehend sichtbar, vom anderen L-förmigen Schlitz ist nur ein Teil seines proximalen Endes erkennbar. Jeder L-förmige Schlitz weist einen axialen Abschnitt 34 und an dessen proximalem Ende einen umfänglichen Abschnitt 35 auf. Die L-förmigen Schlitze 34, 35 sind so angeordnet und ausgebildet, dass sie je eine Knagge 91 an der Schaftkupplung 90 am proximalen Ende einer Gelenkeinrichtung 60 (vgl. Figur 8) und einen Riegel 95 aufnehmen können.

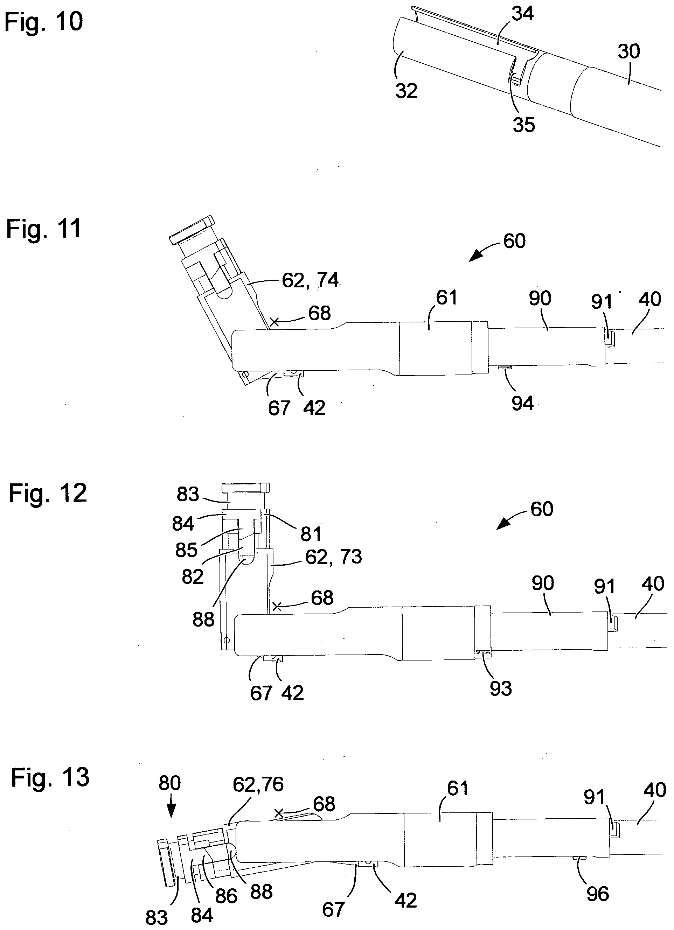

Figur 10 zeigt eine weitere schematische axonometrische Darstellung des distalen Endes 32 des Außenschafts 30 aus Figur 9. Die Darstellung in Figur 10 unterscheidet sich von der Darstellung in Figur 9 durch eine andere Perspektive bzw. einen anderen Betrachterstand.

Zunächst die Wirkung des Riegels 95 (vgl. Figur 8) vernachlässigend, können nach einem Einführen des Innenschafts 40 in den Außenschaft 30 die Knaggen 91 am proximalen Ende der Gelenkeinrichtung 60 durch eine axiale Bewegung von distal nach proximal in die axialen Abschnitte 34 der L-förmigen Schlitze am distalen Ende 32 des Außenschafts 30 eingeführt werden. Wenn die Knaggen 91 die proximalen Enden der axialen Abschnitte 34 der L-förmigen Schlitze erreicht haben, können sie durch eine Rotation der Gelenkeinrichtung 60 relativ zum Außenschaft 30 um die Längsachse 38 (vgl. Figur 1) in die umfänglichen Abschnitte 35 der L-förmigen Schlitze eingeführt werden.

Wenn sich die Knaggen 91 in den umfänglichen Abschnitten 35 der L-förmigen Schlitze befinden, und wenn der Riegel 95 sich in der in Figur 8 gezeigten Position befindet, greifen die Riegel 95 an der Schaftkupplung 90 am proximalen Ende der Gelenkeinrichtung 60 in die axialen Abschnitte 34 der L-förmigen Schlitze ein. Dadurch werden eine Rotation der Gelenkeinrichtung 60 relativ zum Außenschaft 30 verhindert, die Knaggen 91 an der Gelenkeinrichtung 60 in den umfänglichen Abschnitten 35 der L-förmigen Schlitze am distalen Ende 32 des Außenschafts 30 gehalten und so die starre mechanische Verbindung zwischen Gelenkeinrichtung 60 und Außenschaft 30 verriegelt.

Abweichend von den Darstellungen in den Figuren 9 und 10 kann das distale Ende 32 des Außenschafts 30 von einer dünnwandigen kreiszylindermantelförmigen Stützhülse umgeben sein.

Die Figuren 11 bis 13 zeigen weitere schematische Darstellungen der Gelenkeinrichtung 60 aus den Figuren 4 bis 8. In den Figuren 11 bis 13 sind das distale Ende 42 des Innenschafts 40 und der Pleuel 67, der das distale Ende 42 des Innenschafts 40 mit dem distalen Teil 62 der Gelenkeinrichtung 60 koppelt, jeweils teilweise sichtbar.

In den Figuren 11 bis 13 ist erkennbar, dass verschiedene axial-translatorische Positionen des Innenschafts 40 bzw. von dessen distalem Ende 42 zu verschiedenen Winkelpositionen 73, 74, 76 des distalen Teils 62 der Gelenkeinrichtung 60 korrespondieren. Ferner ist erkennbar, dass aufgrund einer starren Kopplung mit dem Innenschaft 40 verschiedene Positionen 93, 94, 96 des Riegels an der Schaftkupplung 90 zu den Winkelpositionen 73, 74, 76 des distalen Teils 62 der Gelenkeinrichtung 60 korrespondieren.

Bei der in Figur 12 gezeigten rechtwinklig abgewinkelten Winkelposition 73 des distalen Teils 62 der Gelenkeinrichtung 60 nimmt der Riegel eine entriegelnde Position 93 im proximalen Ende der Gelenkeinrichtung 61 ein, in der er in einen axialen Abschnitt 34 eines L-förmigen Schlitzes am distalen Ende 32 eines Außenschafts 30 (vgl. Figuren 9 und 10) nicht eingreifen kann. Die in Figur 12 gezeigte Winkelposition 73 des distalen Teils 62 der Gelenkeinrichtung 60 ist somit mit Bezug auf eine mechanische Verbindung der Gelenkeinrichtung 60 mit einem distalen Ende 32 eines Außenschafts 30 mittels der Schaftkupplung 90 eine Löse-Winkelposition.

Mit Bezug auf die mechanische Verbindung der Gelenkeinrichtung 60 mit einem Werkzeug 20 (vgl. Figuren 2 und 3) mittels der Kupplung 80 ist hingegen die in Figur 13 gezeigte Winkelposition 76 des distalen Teils 62 der Gelenkeinrichtung 60 eine Löse-Winkelposition.

Symmetrisch zu dem in den Figuren 8 und 11 bis 13 sichtbaren Riegel 93, 94, 95, 96 kann ein weiterer Riegel in einem weiteren Schlitz vorgesehen sein.

### Bezugszeichen

- 10: Medizinisches Instrument
- 11: proximales Ende des medizinischen Instruments 10
- 12: distales Ende des medizinischen Instruments 10
- 14: Handhabungseinrichtung am proximalen Ende 11 des medizinischen Instruments 10
- 20: Werkzeug
- 21: proximales Ende des Werkzeugs 20
- 22: feststehendes Maulteil des Werkzeugs 20
- 23: schwenkbares Maulteil des Werkzeugs 20
- 25: Knagge am proximalen Ende 21 des Werkzeugs 20
- 26: Hülse am proximalen Ende 21 des Werkzeugs 20
- 28: Rotationsachse des Werkzeugs 20
- 30: Außenschaft
- 31: proximales Ende des Außenschafts 30
- 32: distales Ende des Außenschafts 30
- 34: axialer Abschnitt eines Schlitzes am distalen Ende 32 des Außenschafts 30
- 35: umfänglicher Abschnitt eines Schlitzes am distalen Ende 32 des Außenschafts 30
- 38: Längsachse des Außenschafts 30
- 40: Innenschaft
- 50: Übertragungsstange
- 51: starrer proximaler Abschnitt der Übertragungsstange 50
- 52: elastischer distaler Abschnitt der Übertragungsstange 50
- 60: Gelenkeinrichtung
- 61: proximaler Teil der Gelenkeinrichtung 60
- 62: distaler Teil der Gelenkeinrichtung 60
- 67: Pleuel
- 68: Schwenkachse der Gelenkeinrichtung 60
- 71: Nut am proximalen Teil 61 der Gelenkeinrichtung 60
- 72: Steg am distalen Teil 62 der Gelenkeinrichtung 60
- 73: Löse-Winkelposition des distalen Teils 62 der Gelenkeinrichtung 60
- 74: erste Arbeits-Winkelposition des distalen Teils 62 der Gelenkeinrichtung 60
- 75: zweite Arbeits-Winkelposition des distalen Teils 62 der Gelenkeinrichtung 60
- 76: dritte Arbeits-Winkelposition des distalen Teils 62 der Gelenkeinrichtung 60
- 80: Kupplung am distalen Ende 62 der Gelenkeinrichtung 60
- 81: axialer Abschnitt einer Nut an der Kupplung 80
- 82: umfänglicher Abschnitt einer Nut an der Kupplung 80
- 83: Feder
- 84: Schieber an der Kupplung 80
- 85: Riegel am Schieber 84 in verriegelnder Position
- 86: Riegel am Schieber 84 in entriegelnder Position
- 87: Steg zwischen Riegel 85, 86 und Nocken 88
- 88: Nocken am Schieber 84
- 90: Kupplung am proximalen Ende 61 der Gelenkeinrichtung 60
- 91: Knagge an der Kupplung 90
- 93: Riegel an der Kupplung 90 (entriegelnde Position)
- 94: Riegel an der Kupplung 90 (erste verriegelnde Position)
- 95: Riegel an der Kupplung 90 (zweite verriegelnde Position)
- 96: Riegel an der Kupplung 90 (dritte verriegelnde Position)

## Patentansprüche

1. Gelenkeinrichtung (60) für ein medizinisches Instrument (10), mit:
einer Kupplung (80, 90) zur lösbaren mechanischen Verbindung der Gelenkeinrichtung (60) mit einem Werkzeug (20) oder mit dem distalen Ende (32) eines Schafts (30);
einer Verriegelungseinrichtung (84, 85, 86, 93, 94, 95, 96) an der Kupplung (80, 90), zur Verriegelung der mechanischen Verbindung;
einem Gelenk (71, 72), das ein Abwinkeln der Kupplung (80, 90) relativ zu einem von der Kupplung (80, 90) abgewandten Ende (61) der Gelenkeinrichtung (60) ermöglicht,
**dadurch gekennzeichnet, dass** die Verriegelungseinrichtung (84, 85, 86, 93, 94, 95, 96) so ausgebildet ist, dass die mechanische Verbindung bei allen Arbeits-Winkelpositionen (74, 75) des Gelenks (71, 72) innerhalb eines vorbestimmten Arbeitsbereichs von Arbeits-Winkelpositionen (74, 75) verriegelt und bei einer vorbestimmten Löse-Winkelposition (73, 76) des Gelenks (71, 72) lösbar ist.

2. Gelenkeinrichtung (60) nach dem vorangehenden Anspruch, bei der die Verriegelungseinrichtung (84, 85, 86, 93, 94, 95, 96) ausgebildet ist, um ein Herstellen und Verriegeln der mechanischen Verbindung auch in der Arbeits-Winkelposition (74, 75) des Gelenks (71 ,72) zu ermöglichen.

3. Gelenkeinrichtung (60) nach einem der vorangehenden Ansprüche, bei der die Verriegelungseinrichtung einen in axialer Richtung (28, 38) der Kupplung (80, 90) bewegbaren Riegel (85, 86, 93, 94, 95, 96) umfasst, wobei in einer vorbestimmten verriegelnden Position (85, 94, 95, 96) des Riegels die mechanische Verbindung verriegelt und in einer vorbestimmten entriegelnden Position (86, 93) des Riegels die mechanische Verbindung lösbar ist.

4. Gelenkeinrichtung (60) nach dem vorangehenden Anspruch, bei der
die Kupplung eine Werkzeugkupplung (80) zur lösbaren mechanischen Verbindung der Gelenkeinrichtung (60) mit einem Werkzeug (20) ist,
der Riegel (85, 86) an einem die Kupplung (80) umfassenden distalen Teil (62) der Gelenkeinrichtung (60) angeordnet und geführt ist,
bei der vorbestimmten Löse-Winkelposition (76) des Gelenks (60) der Riegel (86) an einem mit einem Schaft (30) verbundenen oder verbindbaren proximalen Teil (61) der Gelenkeinrichtung (60) anliegt und von diesem in die entriegelnde Position (86) geschoben ist.

5. Gelenkeinrichtung (60) nach dem vorangehenden Anspruch, bei der die Kupplung (80) eine Bajonettkupplung ist und eine Nut (81, 82) zur Aufnahme einer Knagge (25) an einem proximalen Ende (21) eines Werkzeugs (20) umfasst.

6. Gelenkeinrichtung (60) nach einem der vorangehenden Ansprüche, bei der die Kupplung eine Werkzeugkupplung (80) zur lösbaren mechanischen Verbindung der Gelenkeinrichtung (60) mit einem Werkzeug (20) ist, ferner mit:
einer Schaftkupplung (90) zur lösbaren mechanischen Verbindung der Gelenkeinrichtung (60) mit einem Schaft (30),
einer Schaft-Verriegelungseinrichtung (93, 94, 95, 96),
wobei die Schaft-Verriegelungseinrichtung (93, 94, 95, 96) ausgebildet ist, um die mechanische Verbindung der Gelenkeinrichtung (60) mit einem Schaft (30) mittels der Schaftkupplung (90) zu verriegeln.

7. Gelenkeinrichtung (60) nach dem vorangehenden Anspruch, bei der die Schaft-Verriegelungseinrichtung (93, 94, 95, 96) derart mit dem Gelenk (71, 72) gekoppelt ist, dass die Schaftkupplung (90) bei einer Schaftlöse-Winkelposition (73) des Gelenks (71, 72) entriegelt ist.

8. Gelenkeinrichtung (60) nach dem vorangehenden Anspruch, ferner mit:
einer Übertragungseinrichtung (40), die mit dem Gelenk (71, 72) gekoppelt ist, zum Übertragen einer Kraft vom proximalen Ende (11) eines medizinischen Instruments (10) zu dem Gelenk (71, 72), und zum Einstellen einer Winkelposition (73, 74, 75, 76) des Gelenks (71, 72) vom proximalen Ende (11) des medizinischen Instruments (10) aus,
wobei die Schaft-Verriegelungseinrichtung (93, 94, 95, 96) mit der Übertragungseinrichtung gekoppelt ist.

9. Gelenkeinrichtung (60) nach einem der Ansprüche 1 bis 4, bei der
die Kupplung eine Schaftkupplung (90) zur lösbaren mechanischen Verbindung der Gelenkeinrichtung (60) mit einem Schaft (30) ist,
ein Riegel an einem die Kupplung (90) umfassenden proximalen Teil (61) der Gelenkeinrichtung (60) angeordnet und geführt ist,
bei der vorbestimmten Löse-Winkelposition des Gelenks (71, 72) der Riegel an einem distalen Teil (62) der Gelenkeinrichtung (60), der mit einem Werkzeug (20) verbunden oder verbindbar ist, anliegt und von dem distalen Teil (62) der Gelenkeinrichtung (60) in eine entriegelnde Position geschoben ist.

10. Medizinisches Instrument (10) mit einer Gelenkeinrichtung (60) nach einem der vorangehenden Ansprüche.

11. Medizinisches Instrument (10) nach dem vorangehenden Anspruch, ferner mit:
einem Schaft (30), dessen distales Ende (32) mit der Gelenkeinrichtung (60) verbindbar oder verbunden ist;
einer Handhabungseinrichtung (14), die mit dem proximalen Ende (31) des Schafts (30) lösbar mechanisch koppelbar ist,
wobei die vorbestimmte Löse-Winkelposition (73, 76) des Gelenks (71, 72) nicht erreichbar ist, wenn die Handhabungseinrichtung (14) mit dem proximalen Ende (31) des Schafts (30) gekoppelt ist.

## Claims

1. Articulation device (60) for a medical instrument (10), comprising:
a coupling (80, 90) for releasable mechanical connection of the articulation device (60) to a tool (20) or to the distal end (32) of a shaft (30) ;
a locking device (84, 85, 86, 93, 94, 95, 96) on the coupling (80, 90) for locking the mechanical connection;
a hinge (71, 72), which enables the coupling (80, 90) to be angled relative to an end (61) of the articulation device (60) remote from the coupling (80, 90),
**characterized in that** the locking device (84, 85, 86, 93, 94, 95, 96) is designed such that the mechanical connection is locked at all working angular positions (74, 75) of the hinge (71, 72) within a predetermined working range of working angular positions (74, 75) and is releasable at a predetermined release angular position (73, 76) of the hinge (71, 72).

2. Articulation device (60) according to the preceding claim, wherein the locking device (84, 85, 86, 93, 94, 95, 96) is designed so as to enable a closing and locking of the mechanical connection even in the working angular position (74, 75) of the hinge (71, 72).

3. Articulation device (60) according to one of the preceding claims, wherein the locking device comprises a bolt (85, 86, 93, 94, 95, 96) movable in the axial direction (28, 38) of the coupling (80, 90), wherein, in a predetermined locking position (85, 94, 95, 96) of the bolt, the mechanical connection is locked, and, in a predetermined unlocking position (86, 93) of the bolt, the mechanical connection is releasable.

4. Articulation device (60) according to the preceding claim, wherein
the coupling is a tool coupling (80) for releasable mechanical connection of the articulation device (60) to a tool (20),
the bolt (85, 86) is arranged and guided on a distal part (62) of the articulation device (60) comprising the coupling (80),
the bolt (86), in the predetermined release angular position (76) of the articulation (60), bears against a proximal part (61) of the articulation device (60), said proximal part being connected or connectable to a shaft (30), and is slid by said proximal part into the unlocking position (86).

5. Articulation device (60) according to the preceding claim, wherein the coupling (80) is a bayonet coupling and comprises a groove (81, 82) for receiving a catch (25) at a proximal end (21) of a tool (20).

6. Articulation device (60) according to one of the preceding claims, wherein the coupling is a tool coupling (80) for releasable mechanical connection of the articulation device (60) to a tool (20), further comprising:
a shaft coupling (90) for releasable mechanical connection of the articulation device (60) to a shaft (30),
a shaft locking device (93, 94, 95, 96),
wherein the shaft locking device (93, 94, 95, 96) is designed so as to lock the mechanical connection of the articulation device (60) to a shaft (30) by means of the shaft coupling (90).

7. Articulation device (60) according to the preceding claim, wherein the shaft locking device (93, 94, 95 96) is coupled to the hinge (71, 72) in such a way that the shaft coupling (90) is unlocked in a shaft release angular position (73) of the hinge (71, 72).

8. Articulation device (60) according to the preceding claim, further comprising:
a transfer device (40), which is coupled to the hinge (71, 72), for transferring a force from the proximal end (11) of a medical instrument (10) to the hinge (71, 72) and for adjusting an angular position (73, 74, 75, 76) of the hinge (71, 72) from the proximal end (11) of the medical instrument (10),
wherein the shaft locking device (93, 94, 95, 96) is coupled to the transfer device.

9. Articulation device (60) according to one of Claims 1 to 4, wherein
the coupling is a shaft coupling (90) for releasable mechanical connection of the articulation device (60) to a shaft (30),
a bolt is arranged and guided on a proximal part (61) of the articulation device (60) comprising the coupling (90),
the bolt, in the predetermined release angular position of the hinge (71, 72), bears against a distal part (62) of the articulation device (60), said distal part being connected or connectable to a tool (20), and is slid by the distal part (62) of the articulation device (60) into an unlocking position.

10. Medical instrument (10) comprising an articulation device (60) according to one of the preceding claims.

11. Medical instrument (10) according to the preceding claim, further comprising:
a shaft (30), of which the distal end (32) is connectable or is connected to the articulation device (60);
a handling device (14), which can be releasably mechanically coupled to the proximal end (31) of the shaft (30),
wherein the predetermined release angular position (73, 76) of the hinge (71, 72) cannot be reached when the handling device (14) is coupled to the proximal end (31) of the shaft (30).

## Revendications

1. Dispositif d'articulation (60) pour instrument médical (10), présentant :
un accouplement (80, 90) permettant une liaison mécanique libérable entre le dispositif d'articulation (60) et un outil (20) ou l'extrémité distale (32) d'une tige (30),
un dispositif de verrouillage (84, 85, 86, 93, 94, 95, 96) prévu sur l'accouplement (80, 90) pour verrouiller la liaison mécanique,
une articulation (71, 72) qui permet de couder l'accouplement (80, 90) par rapport à une extrémité (61) du dispositif d'articulation (60) non tournée vers l'accouplement (80, 90),
**caractérisé en ce que**
le dispositif de verrouillage (84, 85, 86, 93, 94, 95, 96) est configuré de telle sorte que la liaison mécanique soit verrouillée dans toutes les positions angulaires de travail (74, 75) de l'articulation à l'intérieur d'une plage de travail prédéterminée de positions angulaires de travail (74, 75) et qu'elle puisse être libérée pour une position angulaire de libération (73, 76) prédéfinie de l'articulation (71, 72).

2. Dispositif d'articulation (60) selon la revendication précédente, dans lequel le dispositif de verrouillage (84, 85, 86, 93, 94, 95, 96) est configuré pour permettre d'établir et de verrouiller la liaison mécanique même dans la position angulaire de travail (74, 75) de l'articulation (71, 72).

3. Dispositif d'articulation (60) selon l'une des revendications précédentes, dans lequel le dispositif de verrouillage comporte un verrou (85, 86, 93, 94, 95, 96) apte à se déplacer dans la direction axiale (28, 38) de l'accouplement (80, 90), la liaison mécanique étant verrouillée dans une position prédéterminée de verrouillage (85, 94, 95, 96) et la liaison mécanique pouvant être libérée dans une position prédéterminée (86, 93) de déverrouillage du verrou.

4. Dispositif d'articulation (60) selon la revendication précédente, dans lequel
l'accouplement est un accouplement d'outil (80) permettant de relier mécaniquement de manière libérable le dispositif d'articulation (60) à un outil (20),
le verrou (85, 86) est disposé et guidé sur une partie distale (62) du dispositif d'articulation (60) qui comprend l'accouplement (80) et
le verrou repose sur une partie proximale (61) du dispositif d'articulation (60), reliée ou apte à être reliée à une tige (30), et est déplacé par cette partie dans la position de déverrouillage (86), dans une position angulaire prédéterminée de libération (76) de l'articulation (60).

5. Dispositif d'articulation (60) selon la revendication précédente, dans lequel l'accouplement (80) est un accouplement à baïonnette et comporte une rainure (81, 82) de reprise d'un taquet (25) prévu sur une extrémité proximale (21) d'un outil (20).

6. Dispositif d'articulation (60) selon l'une des revendications précédentes, dans lequel l'accouplement est un accouplement d'outil (80) permettant de relier mécaniquement de manière libérable le dispositif d'articulation (60) à un outil (20), et présentant en outre
un accouplement de tige (90) permettant la liaison mécanique libérable du dispositif d'articulation (60) à une tige (30),
un dispositif (93, 94, 95, 96) de verrouillage de tige, le dispositif (93, 94, 95, 96) de verrouillage de tige étant configuré pour verrouiller la liaison mécanique du dispositif d'articulation (60) à une tige (30) au moyen de l'accouplement de tige (90).

7. Dispositif d'articulation (60) selon la revendication précédente, dans lequel le dispositif (93, 94, 95, 96) de verrouillage de tige est accouplé à l'articulation (71, 72) de telle sorte que l'accouplement de tige (90) soit déverrouillé lorsque l'articulation (71, 72) se trouve dans une position angulaire (73) de libération de tige.

8. Dispositif d'articulation (60) selon la revendication précédente, présentant en outre un dispositif de transfert (40) accouplé à l'articulation (71, 72) et permettant de transférer une force de l'extrémité proximale (11) d'un instrument médical (10) vers l'articulation (71, 72) et pour établir une position angulaire (73, 74, 75, 76) de l'articulation (71, 72) par rapport à l'extrémité proximale (11) de l'instrument médical (10), le dispositif (93, 94, 95, 96) de verrouillage de tige étant accouplé au dispositif de transfert.

9. Dispositif d'articulation (60) selon l'une des revendications 1 à 4, dans lequel l'accouplement est un accouplement de tige (90) permettant une liaison mécanique libérable du dispositif d'articulation (60) à une tige (30),
un verrou étant disposé et guidé sur une partie proximale (61) du dispositif d'articulation (60) qui comporte l'accouplement (90),
le verrou reposant sur une partie distale (62) du dispositif d'articulation (60) qui est relié ou peut être relié à un outil (20) lorsque l'articulation (71, 72) se trouve dans une position angulaire prédéterminée de libération et étant amené dans une position de déverrouillage depuis la partie distale (62) du dispositif d'articulation (60).

10. Instrument médical (10) présentant un dispositif d'articulation (60) selon l'une des revendications précédentes.

11. Instrument médical (10) selon la revendication précédente, présentant en outre :
une tige (30) dont l'extrémité distale (32) peut être reliée ou est reliée au dispositif d'articulation (60),
un dispositif de manipulation (14) qui peut être accouplé mécaniquement de manière libérable à l'extrémité proximale (60) de la tige (30),
la position angulaire prédéterminée de libération (73, 76) de l'articulation (71, 72) ne pouvant être atteinte si le dispositif de manipulation (14) est accouplé à l'extrémité proximale (31) de la tige (30).
